# EUROPEAN PATENT APPLICATION

(11) **EP 1 707 561 A1**
(43) Date of publication of application: **04.10.2006**
(21) Application number: 05703798.8
(22) Date of filing: 19.01.2005
(51) Int. Cl.: C07C 275/40, C07C 271/28, C09K 3/00

(54) **NOVEL TRIPHENYLMETHANE DERIVATIVE, ORGANIC GELLANT CONTAINING THE SAME, ORGANIC GEL, AND ORGANIC FIBER**

(30) Priority: 19.01.2004 JP 2004010306
(71) Applicant: MITSUBISHI GAS CHEMICAL COMPANY, INC., Chiyoda-ku, Tokyo 100-8324 (JP)
(72) Inventor: OGURO,D, Mitsubishi Gas Chem. Company Inc, Hiratsuka-sh i Kanagawa 254-0016 (JP)
(74) Representative: Gille Hrabal Struck Neidlein Prop Roos
(86) International application number: PCT/JP2005/000561
(87) International publication number: WO 2005/068417

(57) **Abstract**

In accordance with the present invention, there are provided a triphenylmethane derivative represented by the following general formula (1), an organic gelling agent containing the triphenylmethane derivative, an organic gel and an organic fiber. The triphenylmethane derivatives of the present invention can exhibit a capability of gelling various organic solvents even when used in a small amount notwithstanding these derivatives are low-molecular compounds. The resultant organic gel is useful as materials usable under a high-temperature condition such as chemomechanical system materials, impact and vibration absorbing materials, drug base materials, controlled drug-release materials, and silicone oil gels for solidification of electrolytic solutions and for cosmetics. In addition, an organic nanofiber can be produced from the triphenylmethane derivative by a simple process. The organic nanofiber can be applied to wiring materials for electronic devices, separation membranes for nano-scale substances, high-efficiency photocatalysts, and culture media for regenerative medical treatments or filters for preventing biochemical hazards utilizing a nonwoven fabric (nano-fabric) made of nanofiber.

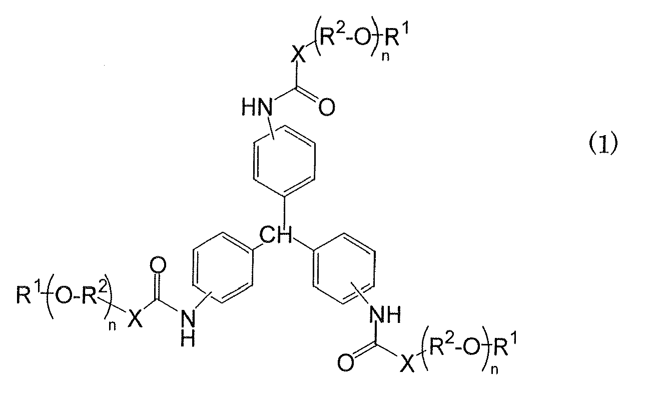

## Description

### TECHNICAL FIELD

The present invention relates to novel triphenylmethane derivatives, organic gelling agents containing the triphenylmethane derivatives, organic gels and organic fibers. The triphenylmethane derivatives have a capability of gelling various organic solvents under heating and readily producing organic nanofibers.

### BACKGROUND ART

A gel means such a substance having no or substantially no fluidity which is obtained by coagulating colloid particles in a colloid solution under a certain condition to form a network structure and enclosing the solution into such a network structure. The gel is generally classified into a hydrogel and an organic gel depending upon whether the solvent as a constituent of the gel is water or an organic solvent.
The organic gel containing, as a constituent thereof, an organic solvent having a boiling point higher than that of water can be used in the applications to which the hydrogel is inapplicable, such as impact and vibration absorbing materials usable under a high temperature condition, sustained drug-release materials, recovering agents for liquid organic materials, and silicone oil gels for solidification of electrolytic solutions and for cosmetics, as well as wiring materials for electronic devices such as metallic nanowires produced by using organic nanofibers obtained from the organic gel as a template, separation membranes for nano-scale substances, high-efficiency photocatalysts, and culture media for regenerative medical treatments or filters for preventing biochemical hazards using a nonwoven fabric (nano-fabric) made of nanofibers.

The organic gel is generally more difficult to produce as compared to the hydrogel. Therefore, only a small number of methods for producing the organic gel have been conventionally reported. For example, in Non Patent Document 1, there is described the method of producing an organic gel from poly(benzyl glutamate) and dioxane. However, in this method, the gelation requires a temperature as high as 70°C and a period as long as 10 to 20 days.
Also, Patent Document 1 discloses the organic gelling agent containing a polyamide constituted from a nitro-containing aromatic dicarboxylic acid and a metal salt such as iron chloride. However, the production of the organic gelling agent requires such a complicated two-stage procedure in which the gelling agent is first uniformly dissolved in a solvent, and then the metal salt as a coagulant is added to the obtained solution. In addition, there tends to arise such a problem that the use of the metal salt causes coloration of the obtained gel and corrosion of containers or equipments used.
Further, Patent Document 2 discloses the organic gelling agent composed of a cyclohexane derivative. However, the organic gelling agent has a minimum gelling concentration of 15 g/L or more and, therefore, may fail to exhibit a sufficient gelling performance.

Patent Document 1: Japanese Patent Application Laid-open No. 95611/1997
Patent Document 2: Japanese Patent Application Laid-open No. 64047/2003
Non Patent Document 1: "Macromolecule", Vol. 23, p. 3779 (1990)

### DISCLOSURE OF THE INVENTION

An object of the present invention is to provide an organic gelling agent which can exhibit a high gelling performance capable of readily gelling an organic solvent without need of adding a secondary component (coagulant) such as a metal salt thereto only by heating the organic gelling agent together with the organic solvent and then allowing the resultant material to stand at room temperature for cooling; an organic gel composed of the organic gelling agent; and an organic filer.

As a result of intensive and extensive researches to solve the above problems, the inventors have found that a novel triphenylmethane derivative having a strong hydrogen bond-forming capability and containing an urea bond therein can exhibit excellent properties as a gelling agent, and an organic fiber having a length of several tens nanometers can be readily produced from a gel obtained using the gelling agent by a simple method. The present invention has been accomplished on the basis of the above finding.

Thus, the present invention provides the triphenylmethane derivative, organic gelling agent, organic gel and organic fiber described in the following aspects [1] to [9]:
[1] A triphenylmethane derivative represented by the general formula (1):

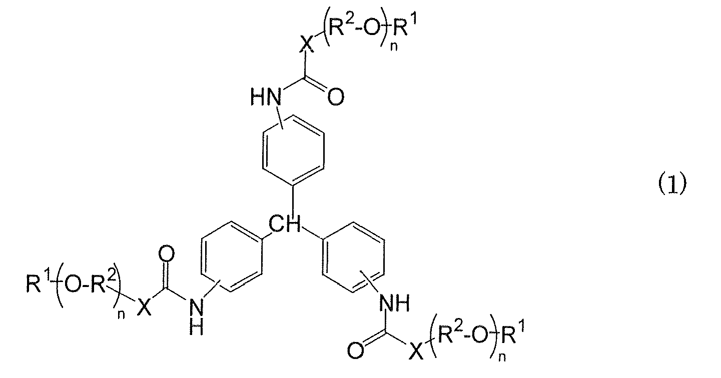

wherein R¹ is a linear or branched alkyl group having 1 to 20 carbon atoms; R² is a linear or branched alkylene group having 2 to 10 carbon atoms; X is NH, NR¹, O or a single bond; n is an integer of 0 to 10; and a plurality of the R¹ groups, the R² groups, the X groups and the integers n may be respectively identical to or different from each other.
[2] The triphenylmethane derivative described in the above aspect [1], wherein the integer n in the general formula (1) is 0 or 1.
[3] The triphenylmethane derivative described in the above aspect [2] which is represented by the general formula (2):

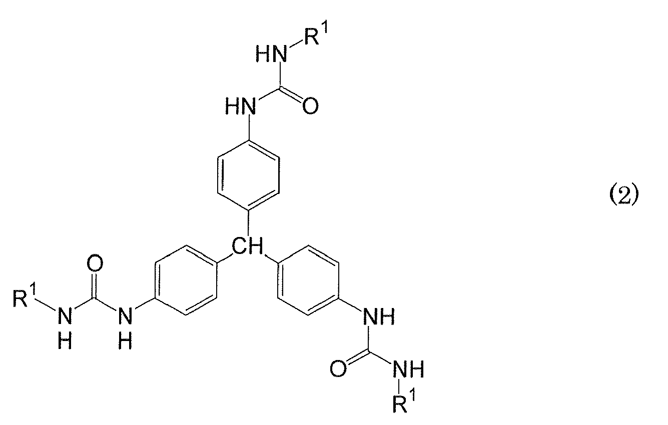

wherein R¹ has the same meaning as defined in the general formula (1).
[4] The triphenylmethane derivative described in the above aspect [3], wherein R¹ is a linear or branched alkyl group having 1 to 5 carbon atoms.
[5] The triphenylmethane derivative described in the above aspect [3], wherein R¹ is a linear or branched alkyl group having 6 to 10 carbon atoms.
[6] The triphenylmethane derivative described in the above aspect [3], wherein R¹ is a linear or branched alkyl group having 11 to 20 carbon atoms.
[7] An organic gelling agent comprising the triphenylmethane derivative described in any one of the above aspects [1] to [6].
[8] An organic gel comprising the organic gelling agent described in the above aspect [7]and an organic solvent.
[9] An organic fiber comprising the organic gel described in the above aspect [8], and having a diameter of 500 nm or less.

### BRIEF DESCRIPTION OF THE DRAWING

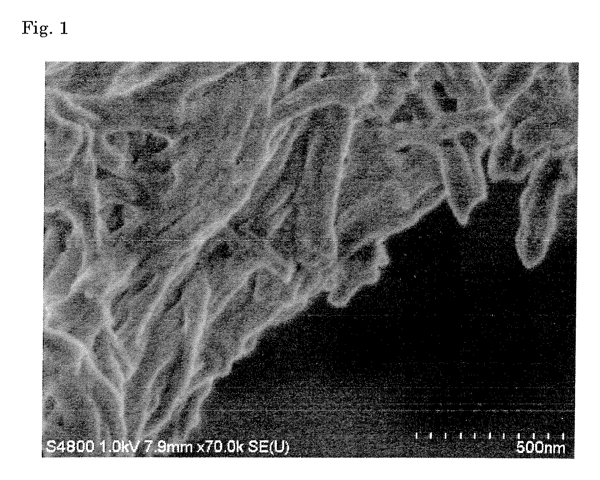

Fig. 1 is an electron photomicrograph showing the organic fiber produced in Example 5.

### PREFERRED EMBODIMENTS FOR CARRYING OUT THE INVENTION

In the triphenylmethane derivatives represented by the general formulae (1) and (2), R¹ is a linear or branched alkyl group having 1 to 20 carbon atoms. Examples of the alkyl group as R¹ include methyl, ethyl, n-propyl, isopropyl, n-butyl, isobutyl, t-butyl, pentyl, hexyl, heptyl, octyl, nonyl, decyl, undecyl, dodecyl, tridecyl , tetradecyl, pentadecyl, hexadecyl, heptadecyl, octadecyl, nonadecyl, eicosadecyl, neopentyl, 2-ethylhexyl, 3,3-dimethylbutyl, 1,1,3,3-tetramethylbutyl, 3,7-dimethyloctyl, 3,7-dimethyloctan-3-yl, 2-hexyldecyl, 2-heptylundecyl, 2-octyldodecyl, 3,7,11-trimethyldodecyl, 3,7,11,15-tetramethylhexadecyl, 3,5,5-trimethylhexyl, 2,3,4-trimethylpentan-3-yl, 2,3,4,6,6-pentamethylheptan-3-yl and isostearyl.
Among these alkyl groups, preferred are n-propyl, n-butyl, t-butyl, hexyl, octyl, decyl, dodecyl, tetradecyl, hexadecyl, heptadecyl, octadecyl, eicosadecyl and neopentyl.
Meanwhile, a plurality of the R¹ groups in the general formulae (1) and (2) may be identical to or different from each other.

In the general formula (1), R² is a linear or branched alkylene group having 2 to 10 carbon atoms; and n is an integer of 0 to 10 and preferably 0 or 1. When n is 2 or more, the R² groups are respectively a linear or branched alkylene group having 2 to 3 carbon atoms, for example, an ethylene group or a propylene group. In the general formula (1), X is NH, NR¹, O or a single bond, and preferably NH. A plurality of the R² groups, a plurality of the integers n and a plurality of the X groups may be respectively identical to or different from each other.
Examples of the linear or branched alkylene group having 2 to 10 carbon atoms as R² include ethylene, propylene, butylene, isobutylene, pentylene, isopentylene, neopentylene, hexylene, cyclohexylene, heptylene, octylene, nonylene and decanylene.
Among the above triphenylmethane derivatives, the compounds represented by the general formula (1) in which n is 0 or 1 are preferred in view of a good availability of raw materials having a triphenylmethane structure, and the compounds represented by the general formula (2), i.e., those compounds of the general formula (1) in which n is 0 and X is NH are more preferred because such compounds having an urea bond exhibit a high gelling performance.

The compounds represented by the general formula (2) in which a plurality of the R¹ groups are each independently a linear or branched alkyl group having 1 to 20 carbon atoms are different in gelling performance from each other depending upon the number of carbon atoms contained therein.
When R¹ is a linear or branched alkyl group having 1 to 5 carbon atoms, the compounds represented by the general formula (2) exhibit a high gelling performance capable of gelling high-polar solvents, for example, propylene carbonate. When R¹ is a linear or branched alkyl group having 6 to 10 carbon atoms, the compounds represented by the general formula (2) exhibit a high gelling performance capable of gelling polar solvents such as isopropanol. Further, when R¹ is a linear or branched alkyl group having 11 to 20 carbon atoms, the compounds represented by the general formula (2) exhibit a high gelling performance capable of gelling hydrophobic solvents such as toluene and decalin. More specifically, the organic gelling agent of the present invention are capable of gelling various polar solvents by varying the kinds of side chains in these compounds.

Next, the process for producing the triphenylmethane derivatives according to the present invention is explained.
The triphenylmethane derivatives represented by the general formula (1) can be produced by reacting triphenylmethane triisocyanate with a compound selected from the group consisting of a monoamine represented by the following general formula (3), a monool represented by the following general formula (4), and a monocarboxylic acid represented by the following general formula (5) or an acid halide thereof in the presence of a solvent such as benzene, toluene, tetrahydrofuran, methylene chloride, ethyl acetate, dimethyl acetamide and N,N-dimethylformamide or in the absence of any solvent, or by reacting triphenylmethane triamine (leuco base) with a monoisocyanate represented by the following general formula (6) or the monocarboxylic acid represented by the following general formula (5) or the acid halide thereof in the presence of the above solvent or in the absence of any solvent. The concentration of the reaction solution is preferably from 1 to 90% and more preferably from 5 to 50%.

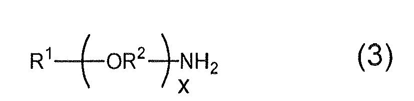

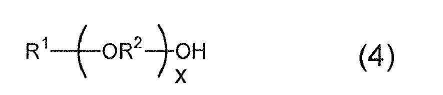

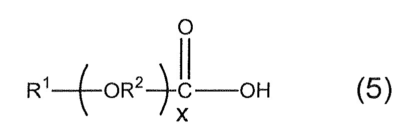

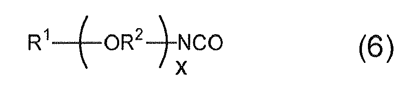

These reactions may be conducted at room temperature. However, the reaction solution may be usually heated to a boiling point of the solvent or in the vicinity of the boiling point. The reaction time is about 30 min to 10 h and preferably about 1 to 5 h. The reaction may be monitored by measuring IR spectra of the reaction solution to observe an absorption peak of an isocyanate group thereof. In this case, the terminal point of the reaction may be determined by dissipation of the isocyanate group. Also, in some cases, a basic catalyst such as triethylamine and pyridine or a metal-containing catalyst such as alkyl tin hydroxide and alkyl titanate may be added to the reaction solution in an amount of 0.001 to 10% by mass in order to shorten the reaction time. The aimed product may be recovered from the reaction mixture, for example, by filtering a precipitate obtained by adding a poor solvent to the reaction mixture, by filtering a precipitate from the reaction mixture directly or after concentrating the mixture under reduced pressure, or by once dissolving the concentrated product obtained under reduced pressure in the other solvent such as alcohols and acetone, cooling the resultant solution to form a precipitate and then filtering the obtained precipitate. The thus recovered precipitate may be dried, if required.

In the above process for producing the triphenylmethane derivatives represented by the general formula (1), when an alkyl alcohol or an alkylcarboxylic acid is used as the raw material, there may be used a basic catalyst such as triethylamine and pyridine, or a metal-containing catalyst such as alkyl tin hydroxide and alkyl titanate. The catalyst is used in the reaction solution in an amount of preferably 0.001 to 10% by weight and more preferably 0.1 to 5% by weight.

Meanwhile, the monool compound represented by the above general formula (4) may be synthesized by reacting a monovalent alcohol as a reaction initiator with ethyleneoxide or propyleneoxide.
Also, the monoamine compound represented by the above general formula (3) may be synthesized by replacing a hydroxyl end group of the monool compound represented by the general formula (4) with an amino group. Examples of the monoamine compound include commercial products "JEFERMINE M Series" available from Hantzman Corp.
The monocarboxylic acid compound represented by the above general formula (5) may be synthesized by oxidizing a hydroxyl end group of the monool compound represented by the above general formula (4).
The monoisocyanate compound represented by the above general formula (6) may be synthesized by replacing an amino end group of the monoamine compound represented by the above general formula (3) with an isocyanate group.

The triphenylmethane derivatives represented by the general formula (1) in which n is 0 or 1 may be produced, for example, by reacting triphenylmethane triisocyanate with an alkyl amine containing an alkyl group having 1 to 20 carbon atoms in the presence of a solvent such as benzene, toluene, tetrahydrofuran, methylene chloride, ethyl acetate, dimethyl acetamide and N,N-dimethylformamide or in the absence of any solvent, or by reacting triphenylmethane triamine (leuco base) with an alkyl isocyanate containing an alkyl group having 1 to 20 carbon atoms in the presence of the above solvent or in the absence of any solvent. The concentration of the reaction solution is preferably from 1 to 90% by mass and more preferably from 5 to 50% by mass.
Meanwhile, the triphenylmethane derivatives of the present invention are preferably produced from triphenylmethane triisocyanate used as a hardening agent for paints in view of a good availability of the raw material.

The triphenylmethane derivatives of the present invention are such compounds having a triphenylmethane structure, and at least one bond selected from the group consisting of an urea bond, an urethane bond and an amino bond.
The triphenylmethane structure is rigid, but tend to hardly undergo crystallization owing to radially extending side chains thereof and, therefore, is susceptible to self-organization due to hydrophobic interaction therebetween. In addition, the urea bond, urethane bond and amino bond exhibit a large hydrogen bonding property and, therefore, tends to form a macro-fibrous associated product owing to an intermolecular effect therebetween.

The triphenylmethane derivatives represented by the general formula (1) of the present invention have a capability of forming a network structure in the presence of an organic solvent and are therefore useful as an organic gelling agent. The network structure formed by the triphenylmethane derivatives of the present invention is different from those of high-molecular compounds or inorganic compounds, and can be retained only by a non-conjugated bond such as a hydrogen bond. The process starting from the single molecule and finally terminating at formation of the network structure composed of entangled fibrous associated products, i.e., self-agglomeration thereof, is a heat reversible process.
Also, the triphenylmethane derivatives of the present invention are swelled upon mixing with the organic solvent to form an organic gel. Upon forming the organic gel, the amount of the triphenylmethane derivative on the basis of the organic solvent varies depending upon the kind of the organic solvent used, and is usually from 1 to 50 g/L and preferably from 2 to 15 g/L. The gelling temperature may be selected from the range of from room temperature to the boiling point of the organic solvent.

The organic fiber of the present invention is produced from the above organic gel. For example, the organic fiber may be produced by freeze-drying the organic gel, or by immersing the organic gel in a poor solvent having a low dissolvability to the organic gelling agent for 6 h or longer and then drying the resultant gelled material. The poor solvent used varies depending upon a polarity of the organic gelling agent used. The organic fiber of the present invention has a diameter of 500 nm or less, preferably 200 nm or less and more preferably 100 nm or less.

### EXAMPLES

The present invention is described in more detail by referring to the following examples. However, it should be noted that these examples are only illustrative and not intended to limit the invention thereto.
Meanwhile, the gelling performance test for triphenylmethane derivatives obtained in the respective Examples was conducted by the following method.

### <Gelling performance test>

A test tube with a lid was charged with 0.01 g of the compound (triphenylmethane derivative). After adding 1 mL of an organic solvent to the test tube, the contents of the test tube were heated to dissolve the compound in the organic solvent. The resultant solution was allowed to stand at room temperature (25°C) for 30 min, and observed to examine whether any gelation of the solution having a concentration of 10 g/L was caused or not. As to the gelled solution, a minimum gelling concentration (g/L) thereof was determined. More specifically, samples of the compound having different weights lower than 0.01 g were accurately weighed and charged into the respective test tubes with a lid, and 1 mL of the organic solvent was added to the respective test tubes. The obtained mixtures were heated at a temperature of 80 to 200°C for 1 min to dissolve the respective samples in the organic solvent, thereby preparing solutions having a reduced gelling concentration. Then, the thus obtained solutions were allowed to stand in a constant-temperature oven maintained at 25°C. After 30 min, the respective test tubes were tilted and lightly shaken to examine the condition of the obtained products therein. Among the products in the respective test tubes, those which were free from oozing of the solution upon tilting and from breaking upon light shaking were regarded as a gel, and a minimum gelling concentration (g/L) thereof was measured. Meanwhile, the test results are shown in Table 1 in which those showing no gelling performance were indicated by the symbol "×" and those subjected to no gelling performance test were indicated by the symbol "―".

### EXAMPLE 1

A reaction vessel was charged with 10 mL of a dried methylene chloride solution containing 2.96 g of stearyl amine (reagent available from Kanto Kagaku Co., Ltd.), and a solution prepared by adding 10 mL of dimethyl acetamide (DMAc) to 4.83 g of an ethyl acetate solution containing 27% by weight of triphenylmethane triisocyanate (TPMTI) ("DISMODULE RE" (tradename) available from Sumitomo Bayer Urethane Co., Ltd.; NCO equivalent: 441) was slowly dropped into the reaction vessel using a dropping funnel, and stirred at room temperature for 1 h. The termination of the reaction was confirmed by dissipation of the NCO group observed at 2230 cm⁻¹ in IR spectra (KBr method). The resultant reaction mixture was added to a large amount of distilled water to obtain a precipitate. The thus obtained precipitate was separated and purified by column chromatography to obtain an aimed product. As a result, it was confirmed that the amount of the aimed product produced was 10.0 g (yield: 90%). The results of the gelling performance test for the obtained product are shown in Table 1.
Elemental Analysis Values (as C₇₆H₁₃₀N₆O₃)
CHN theoretical values (%): 77.6; 11.1; 7.2
CHN measured values (%): 78.1; 11.0; 7.4 As a result of the IR analysis, it was confirmed that an absorption peak at 2231 cm⁻¹ derived from NCO (isocyanate group) was dissipated, whereas an absorption peak at 1639 cm⁻¹ derived from urea was observed. From the raw materials charged as well as the results of the elemental analysis and the IR analysis, it was determined that the obtained product was the compound having a chemical structure represented by the following formula (7):

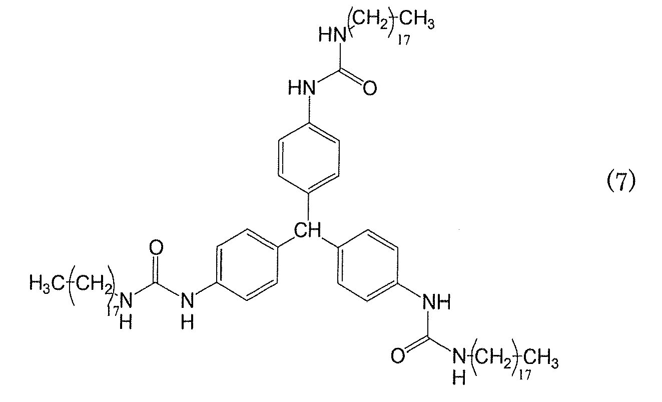

### EXAMPLE 2

The same procedure as in Example 1 was repeated except that stearyl amine was replaced with 4.35 g of octylamine available from Kao Corp. The results of the gelling performance test are shown in Table 1.
Elemental Analysis Values (as C₄₆H₇₀N₆O₃)
CHN theoretical values (%): 73.2; 9.3; 11.1
CHN measured values (%): 73.1; 9.4; 11.0
As a result of the IR analysis, it was confirmed that an absorption peak at 2231 cm⁻¹ derived from NCO was dissipated, whereas an absorption peak at 1636 cm⁻¹ derived from urea was observed.
From the raw materials charged as well as the results of the elemental analysis and the IR analysis, it was determined that the obtained product was the compound having a chemical structure represented by the following formula (8):

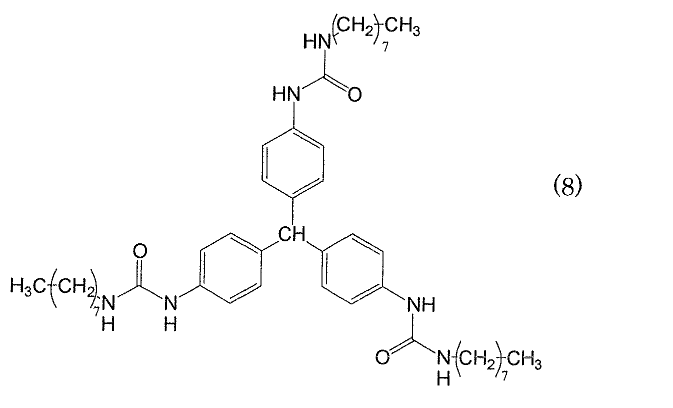

### EXAMPLE 3

The same procedure as in Example 1 was repeated except that stearyl amine was replaced with 4.35 g of n-butylamine as a reagent available from Kanto Kagaku Co. Ltd. The results of the gelling performance test are shown in Table 1.
Elemental Analysis Values (as C₃₄H₄₆N₆O₃)
CHN theoretical values (%): 69.6; 7.9; 14.3
CHN measured values (%): 69.8; 7.8; 14.1
As a result of the IR analysis, it was confirmed that an absorption peak at 2231 cm⁻¹ derived from NCO was dissipated, whereas an absorption peak at 1637 cm⁻¹ derived from urea was observed.
From the raw materials charged as well as the results of the elemental analysis and the IR analysis, it was determined that the obtained product was the compound having a chemical structure represented by the following formula (9):

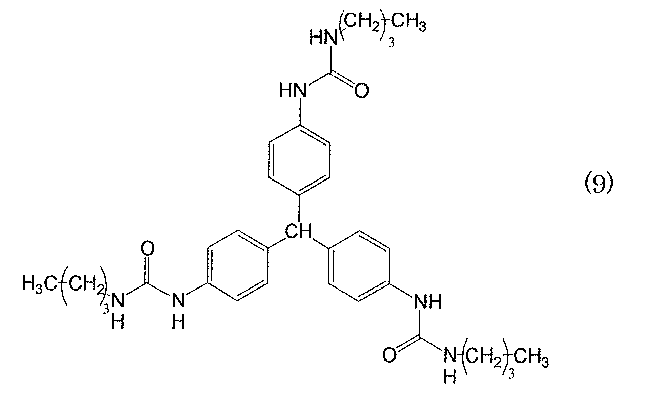

**TABLE 1**

| Organic solvent | Minimum gelling concentration (g/L) | | |
|---|---|---|---|
| | Example 1 | Example 2 | Example 3 |
| Toluene | 2 | × | - |
| 1,1,2,2-tetrachloroethane | 4 | - | - |
| Decalin | 2 | × | × |
| 2-propanol | × | 5 | × |
| Benzonitrile | × | 5 | × |
| Propylene carbonate | × | × | 5 |

In Table 1, the obtained gel was transformed, upon re-heating, into a sol (fluidizable liquid), and further transformed again, upon cooling, into a gel.
From the test results, it was confirmed that although the triphenylmethane derivatives of the present invention were low-molecular organic compounds, the derivatives exhibited a capability of gelling several kinds of organic solvents by changing the kinds of substituent groups thereof without using the other coagulants.

### EXAMPLE 4

A 500 mL egg-shaped flask was charged with 200 g (2.7 mol) of tert-butanol, 100 g (0.625 mol) of 1,9-nonanediol and 2 g of sulfuric acid, and the contents of the flask were refluxed under heating for 5 h and then neutralized with an aqueous sodium hydroxide solution. After distilling off unreacted tert-butanol from the reaction mixture, hexane was added to the reaction mixture, followed by stirring. The hexane-insoluble diol was removed from the reaction mixture by filtration, and the obtained hexane phase was washed with water and then dried with anhydrous sodium sulfate. After filtering the reaction solution and distilling off the solvent therefrom, the resultant residue was purified by silica gel column chromatography to obtain 9-tert-butoxy-1-nanol.
Next, the same procedure as in Example 1 was repeated except that stearylamine was replaced with 4.05 g of 9-tert-butoxy-1-nanol, and 1000 ppm of dibutyl tin dilaurate was added as a catalyst.
Elemental Analysis Values (as C₆₁H₉₇N₃O₉)
CHN theoretical values (%): 72.1; 9.6; 4.1
CHN measured values (%): 72.0; 9.5; 4.3
As a result of the IR analysis, it was confirmed that an absorption peak at 2231 cm⁻¹ derived from NCO was dissipated, whereas an absorption peak at 1620 cm⁻¹ derived from urethane was observed.
From the raw materials charged as well as the results of the elemental analysis and the IR analysis, it was determined that the obtained product was the compound having a chemical structure represented by the following formula (10). Meanwhile, in the following chemical formula (10), the symbol "+" represents a tert-butyl group.

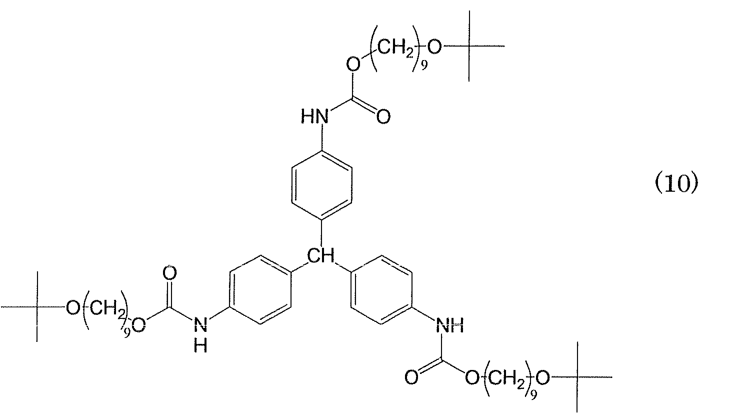

### EXAMPLE 5: Production of Organic Fiber

A test tube with a lid was charged with 0.01 g of the compound obtained in Example 1 (triphenylmethane derivative) and then with 1 mL of 1,1,2,2-tetrachloroethane, and the contents of the test tube were heated and dissolved. The resultant solution was allowed to stand at room temperature (25°C) for 30 min, and the obtained organic gel was immersed in methanol as a poor solvent therefor for 6 h to replace the solvent of the gel with methanol. The thus obtained white gelled product was dried under heating in an oven at 60°C for 1 h, thereby obtaining a white solid. As a result of observing the white solid using a field emission-type scanning electron microscope, it was confirmed that the white solid was an organic fiber having a diameter of about 50 to 150 nm as shown in Fig. 1.

### INDUSTRIAL APPLICABILITY

The triphenylmethane derivatives of the present invention can exhibit a capability of gelling an organic solvent such as toluene, decalin, 1,1,2,2-tetrachloroethane, 2-propanol and propylene carbonate even when used in a small amount notwithstanding these derivatives are low-molecular compounds. The resultant organic gel is useful as materials usable under a high-temperature condition such as chemomechanical system materials, impact and vibration absorbing materials, drug base materials, controlled dryg-release materials, and silicone oil gels for solidification of electrolytic solutions and for cosmetics.
In addition, an organic nanofiber can be produced from the triphenylmethane derivatives of the present invention by a very simple process. The organic nanofiber obtained from the triphenylmethane derivatives can be applied to wiring materials for electronic devices such as metallic nanowires produced by using the organic nanofiber as a template, separation membranes for nano-scale substances, high-efficiency photocatalysts, and culture media for regenerative medical treatments or filters for preventing biochemical hazards utilizing a nonwoven fabric (nano-fabric) made of nanofiber.

## Claims

1. A triphenylmethane derivative represented by the general formula (1): 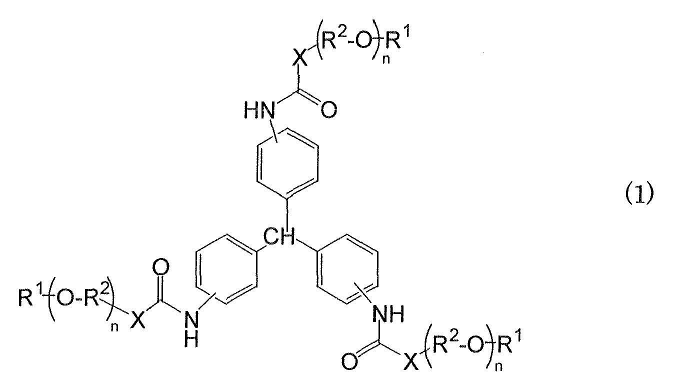 wherein R¹ is a linear or branched alkyl group having 1 to 20 carbon atoms; R² is a linear or branched alkylene group having 2 to 10 carbon atoms; X is NH, NR¹, O or a single bond; n is an integer of 0 to 10; and a plurality of the R¹ groups, the R² groups, the X groups and the integers n may be respectively identical to or different from each other.

2. The triphenylmethane derivative according to claim 1, wherein the integer n in the general formula (1) is 0 or 1.

3. The triphenylmethane derivative according to claim 2 which is represented by the general formula (2): 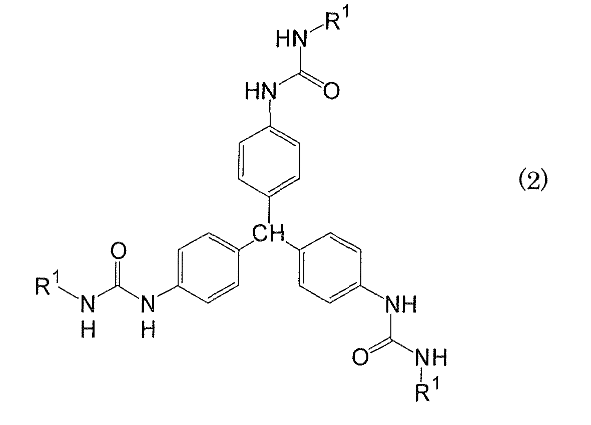 wherein R¹ has the same meaning as defined in the general formula (1).

4. The triphenylmethane derivative according to claim 3, wherein R¹ is a linear or branched alkyl group having 1 to 5 carbon atoms.

5. The triphenylmethane derivative according to claim 3, wherein R¹ is a linear or branched alkyl group having 6 to 10 carbon atoms.

6. The triphenylmethane derivative according to claim 3, wherein R¹ is a linear or branched alkyl group having 11 to 20 carbon atoms.

7. An organic gelling agent comprising the triphenylmethane derivative as defined in any one of claims 1 to 6.

8. An organic gel comprising the organic gelling agent as defined in claim 7, and an organic solvent.

9. An organic fiber comprising the organic gel as defined in claim 8, and having a diameter of 500 nm or less.
